# EUROPEAN PATENT APPLICATION

(11) **EP 1 803 454 A1**
(43) Date of publication of application: **04.07.2007**
(21) Application number: 05028738.2
(22) Date of filing: 30.12.2005
(51) Int. Cl.: A61K 31/277, A61K 38/09, A61N 5/04

(54) **Treatment of cancer by a combination of non-ionizing radiation and androgen deprivation**

(71) Applicant: Dornier MedTech Laser GmbH, 82234 Wessling (DE)
(72) Inventor: Koshiba, Ken, Tokyo 114-0014 (JP)
(74) Representative: Grünecker, Kinkeldey, Stockmair & Schwanhäusser Anwaltssozietät

(57) **Abstract**

The present invention relates to a combined androgen deprivation and thermotherapy for treating cancer. A significant reduction in cancer volume was observed when the androgen deprivation therapy was applied before the radiation treatment started. In all patients treated, no evidence of recurring cancer was observed.

## Description

The present invention relates to a new regimen of treating cancer.

### Background of the Invention

At the beginning of the last century, cancer was the No. 7 cause of disease-based human death. By the end of the 1990s, it rose to No. 2. It is expected that by 2005, cancer will be the number one cause of disease-based death. According to official estimations for Germany, 210,000 people died from cancer in 1997 and 338,000 people developed cancer in the same year.

Cancers differ significantly from each other. Some cancers grow fairly slowly; others rather rapidly. Some produce metastases, others do not. Some form solid tumors, whereas others; like leukemia, do not. The most frequently occurring forms of cancer are: carcinoma of the mamma, lung, stomach and prostate. Different types of cancer not only differ in their biology but also in their treatment regimen. Among the different types of cancer, carcinomas are the most-targeted in cancer therapy.

There are three major approaches in cancer treatment: surgery, radiation and chemotherapy. Often times a combination of the different approaches may be of advantage. For example, tumor size may be first reduced by radiation and/or chemotherapy, and the tumor may subsequently be resected in surgery. Alternatively, tumor size may be first reduced by surgery and then further controlled or reduced by radiation and/or chemotherapy. In the chemotherapeutic approach, compounds that slow down or stop the growth of cells are frequently used. Such compounds are usually cytotoxic and destroy the cells. Radiation therapy and chemotherapy are marred by side effects as neither treatment is highly selective. It is challenging to obtain chemotherapeutic agents that selectively act on cancer cells but not on normal cells. It is also difficult to target radiation selectively to cancer cells but not normal cells.

The above-described three major approaches are often supplemented by further measures such as the administration of hormones, the stimulation of the immune system, and the use of further adjuvants.

Although many treatment regimens are already known in the art, there is still a need for improving existing treatment regimens in order to reduce the side effects and/or to increase the efficiency of cancer destruction.

Observations on the therapeutic effect of heat on malignant tumors have been reported since the early ages. There are many reports in the early 1900s on the use of hyperthermia in treating cancer; and heat was thought to be the most important single factor in causing the regression of the tumors and even the cure of the patients *(*Rohdenburg GL. J. Cancer Res. 1917, 51:19-28*).* Hyperthermia in the early ages consisted mostly of incidental whole body hyperthermia caused by infectious diseases and high body temperature artificially induced with bacterial toxin. However, interest in the use of thermotherapy for treating cancer declined by the 1960s possibly due to the disappearance of many infectious diseases with the introduction of antibiotics and technical difficulties associated with the practical application of hyperthermia.

As used herein, "thermotherapy" is synonymous with "thermoablation" and refers to the process of destroying tissues or cells using thermal energy.

The use of localized hyperthermia for treating prostate cancer using a microwave applicator was reported by Mendecki et al. in 1980 *(*Mendecki J, Friedenthal E, Botstein C. Int. J. Radiation Oncology Biol. Phys. 1980; 6:1583-8). Similar clinical trials have been reported by several groups by the late 1980s *(*Szmigielski S., Zielinski H., Stawarz B. et al. Urol. Res. 1998, 16:1-7*;* Servadio C, Leib Z., Prostate 1984, 5:205-11 Yerushalmi A, Servadio C, Leib Z et al., Prostate 1982; 3: 623-30*,* Roehrborn CG, Preminger G, Newhall P et al., Urology 1998; 51:19-28*).* However, the clinical efficacy was low and the treatment remained supplementary to radiotherapy and/or hormone therapy. The low efficacy was mainly due to the use of the transrectal route which only allowed an intraprostatic temperature of lower than 43 °C to be reached.

A new system, transurethral microwave thermotherapy (TUMT) *(*Devonec M., Tamura K., Perrin P J. Endourol. 1993, 7:255-9), combines the heating of the prostatic tissue with the conductive cooling of the urethra, usually accomplished by arranging a chamber around the microwave antenna which is continuously perfused with cooled water. This urethral applicator reduces the temperature in the immediately adjacent tissue, allowing an even higher power to be used to create sufficient heat deep inside the prostatic tissue, while leaving the temperature of the urethral mucosa and the rectal wall within the safety range. It is generally accepted that microwave treatment can destroy prostatic tissue up to a radial distance of 16 mm, while maintaining innocuous urethral and rectal temperatures. Moreover, temperatures of 45 °C or higher for approximately 1 hour cause uniform thermoablation of the prostatic tissue *(*Larson TR, Bostwick DG, Corica A. Urology 1996, 47:463-9*;* Huidobro C, Balmsjo M, Larson T et al. J. Urol. 2004, 171:672-8).

Regarding the treatment of prostate cancer with TUMT, there are only a few reports up to the present *(*KhairAA, Pacelli A, Iczkowski KA et al. Urology 1999, 54:67-72; Larson BT, Bostwick DG, Corica AG, Larson TR. J. Urol. 2003, 170:12-9*).* They conclude that the effectiveness of TUMT for treating prostate cancer is disappointing mainly because of "the limitation of TUMT in reaching the peripheral prostate." In the majority of their patients, TUMT was done shortly before radical prostatectomy without neoadjuvant hormone therapy (NHT), and the mean volume of the prostate at the time of TUMT exceeded 53 ml which appeared too large to be treated by TUMT only. The radial distance from the urethral wall to the external margin of the fibrous capsule of the prostate with the volume of around 30 ml is usually within 15 mm, with an 18F microwave applicator placed in the urethra.

Adenocarcinoma is the major type of malignant tumors of the prostate; it is an androgen-dependent cancer. Androgen deprivation therapy (ADT) is specifically designed for this cancer and is more effective than any other chemotherapies.

Transitional cell carcinoma and sarcoma are other types of malignant tumors that originate from the prostate gland; they constitute less than 5% of malignant tumors of the prostate. ADT is not effective on these two types of malignant tumors. The term "prostate cancer" used herein would refer to "adenocarcinoma of the prostate".

Initially, bilateral orchiectomy (castration) was done as a hormone therapy to treat prostate cancer; later on estrogen (female hormone) was used. However, estrogen is notorious for its complications to cause cardiovascular insufficiency and liver dysfunction.

Use of LH-RH (luteinizing hormone-releasing hormone) agonist has the same effect as bilateral orchiectomy. LH-RH is produced by the hypothalamus and stimulates the pituitary gland to secrete LH which stimulates the production of testosterone in the testes. However, excess dosage of LH-RH agonist causes suppression of LH secretion, which leads to medical castration. Less than 5% of the androgens are produced by the adrenal glands. These androgens can be blocked by the use of non-steroidal anti-androgens before these androgens are converted to dihydrotestosteron (DHT, the active species) in the prostate. The non-steroidal anti-androgens have fewer side effects than the steroidal anti-androgens.

ADT is synonymous with maximum androgen blockade (MAB), complete androgen blockade (CAB) and total androgen blockade (TAB).

ADT has been used before and after radiation therapy and prostatectomy in the treatment of prostate cancer. However, its effectiveness has been limited. It has been shown that reduction of androgen to castration levels by ADT reduces the size of the prostate and the tumor; when used before and during radiation therapy, it improves local control *(*Pilepich MV, et al. Int. J. Radiat. Oncol. Biol. Phys. 1995, 32:175-80*)* and reduces complications *(*Zelefsky MJ, et al. Urology 1997, 49:38-45*).* However, overall survival of the treated patients did not improve significantly, except in a single trial (The European Organization for Research and Treatment of Cancer multicenter randomized trial, Bolla M, et al. New Eng. J. Med. 1997, 337:295-300*).*

ADT has also been used before radical prostatectomy, a procedure termed neoadjuvant androgen deprivation or neoadjuvant hormone therapy (NHT). NHT has been shown to be an effective but palliative form of therapy *(*Cher ML, et al. Brit. J. Urol. 1995, 75: 771-777*;* Aus G, Abrahamsson PA, et al. BJU International 2002, 90: 561-6*).*

Therefore, one object underlying the present invention was to provide further means for treating cancer, in particular, solid tumor-forming carcinomas.

### Summary of the Invention

The object of the present invention is achieved by using a non-steroidal anti-androgen and/or an LH-RH agonist (i.e., ADT) in combination with the application of non-ionizing radiation, wherein the chemical compounds are administered before, simultaneously with, and/or after the application of the non-ionizing radiation.

The present invention thus provides a therapeutic combination for treating cancer, comprising: (a) a non-steroidal anti-androgen, and/or (b) an LH, LH-RH, or an LH-RH agonist, and (c) non-ionizing radiation, wherein the androgen and/or the agonist are for administration before, simultaneously with, and/or after the application of radiation to the patient. In the following, the use of the term "radiation" means "non-ionizing radiation", if not expressedly stated differently.

In one embodiment, the type of cancer to be treated is a carcinoma. In a preferred embodiment, the cancer to be treated is an adenocarcinoma. In a further preferred embodiment, the cancer to be treated is prostate adenocarcinoma.

"Non-steroidal anti-androgen" used herein refers to an agent which blocks the action of dihydrotestosterone (DHT), a compound that is structurally similar to testosterone and that stimulates protein synthesis in prostate cells and prostate cancer cells. In addition, such an agent would not have any steroidal effects. The non-steroidal anti-androgen is assumed to block the conversion of androgens produced by the adrenal glands to DHT in the prostate and is preferably selected from compounds such as bicalutamide and flutamide.

In a further preferred embodiment the anti-androgen is administered in a dosage of between 10 and 1,000 mg per day. In an even more preferred embodiment, bicalutamide is administered 80-150 mg per day; flutamide is administered 250-375 mg per day.

"LH-RH agonist" used herein refers to a drug that is a synthetic analog of luteinizing hormone-releasing hormone (LH-RH) which is produced in the hypothalamus. LH-RH agonists mimic the function of LH-RH in triggering the pituitary to produce luteinizing hormone (LH), which in turn stimulates the production of testosterone. Because of the higher potency of the agonists, an excess of testosterone is initially produced. The body detects this excess of testosterone and responds by decreasing the production of LH-RH. Thus, after an initial increase in testosterone levels, the LH-RH agonist is assumed to down-regulate the production of testosterone in the testes in excess dosage. An LH-RH agonist is preferably selected from compounds such as leuprorelin acetate, goserelin acetate, buserelin acetate and tripterelin.

The preferred dosage range for the agonist lies in the range of 1-10 mg every 4 weeks. In a further preferred embodiment, leuprorelin acetate is administered 3.75 mg every 4 weeks; goserelin acetate is administered 3.6 mg every 4 weeks. In a more preferred embodiment, leuprorelin is administered 11.25 mg every 3 months; goserelin acetate is administered 10.8 mg every 3 months.

In a preferred embodiment, the treatment with the non-steroidal anti-androgen and the LH-RH agonist (i.e., ADT) is performed for at least about one month, preferably for at least about three months before the application of radiation is started.

In another preferred embodiment, the anti-androgen and the agonist are to be administered sequentially, whereas the anti-androgen is administered first followed by the administration of the LH-RH agonist. In a more preferred embodiment, the non-steroidal anti-androgen is started first; after about one further week, preferably about two further weeks, the LH-RH agonist is administered. However, both compounds may also be administered simultaneously.

The non-steroidal anti-androgen and the LH-RH agonist may be administered by the same route. But as a preferred mode of administration, the non-steroidal anti-androgen is orally administered, whereas the LH-RH agonist is subcutaneously administered.

Diethylstilbestrol (DES) may be administered when ADT becomes ineffective, at a dosage of 100-500 mg per day, either orally or intravenously. DES is a synthetic analog of the female hormone estrogen. Administration of DES increases the level of sex hormones in the body. When the body detects the excess of sex hormones, it regulates the level of such hormones by stopping production of testosterone. This eventually leads to slower growth of prostate cancer cells.

In a further preferred embodiment, the application of the radiation starts at least about four weeks after the start of the treatment with the anti-androgen and/or the agonist (i.e., ADT). More preferably, there are at least about 3 months between the start of the ADT and the radiation.

In a more preferred embodiment, the radiation is applied simultaneously with the ongoing treatment with the anti-androgen and the agonist (i.e. ADT). Simultaneously means that over the period of time where the chemicals are administered, the radiation is also applied. However, the chemicals and the radiation do not need to be administered/applied at the same time point of a day.

In an even more preferred embodiment, the treatment with the anti-androgen and the agonist (i.e. ADT) continues after the radiation, preferably, for about 1 month, and more preferably for about 3 months.

In a preferred embodiment, the radiation to be applied is heat-generating radiation. Thermal energy can be supplied to the tissue to be treated in the form of heat from the local absorption of applied microwave energy, radio frequency energy, or light energy, including energy from laser light.

In a further preferred embodiment the microwave radiation to be administered is in the range of about 800 to about 1500 MHz; more preferably, in the range of about 800 to about 1300 MHz; and even more preferably, in the range of about 800 to about 1000 MHz. It is preferably administered to the target tissue, such as the prostate, via a helical coil antenna which, when applied to the prostate, may be enclosed in a transurethral delivery system, which further may contain a water-cooling circuit system.

In a further preferred embodiment the energy to be administered to the target tissue is within the range of about 25 to about 100 watts; more preferably, in the range of about 25 to about 90 watts; and even more preferably, in the range of about 25 watts to about 85 watts. In a further preferred embodiment the radiation is to be applied by way of transurethral microwave thermoablation (TUMT).

In a further preferred embodiment the radiation is applied over a period of at least about one month, preferably over a period of at least about three months, most preferably, simultaneously with the administration of the antiandrogen and/or the agonist. Simultaneously means that over the period of time where the chemicals are administered, the radiation is also applied. However, the chemicals and the radiation do not need to be administered/applied at the same time point of a day.

### Detailed Description of the Invention

ADT employed before radiation is expected to reduce the total volume of the prostate in the majority of the patients, rendering TUMT effective down to the peripheral prostate. Maximum reduction in prostate volume is expected in 3 months of ADT. Furthermore, 3 months of ADT may reduce the viability of the cancer cells, rendering TUMT more effective.

ADT employed after radiation is expected to accelerate apoptosis of remaining cancer cells which have been weakened by preceding ADT and TUMT Transurethral resection of the prostate (TURP) in radical fashion 3 months after TUMT is expected to reveal the effectiveness of the ADT-radiation combination therapy.

A number of minimally invasive treatments have been developed in recent years for treating benign prostatic hyperplasia (BPH). Some of these new treatments employ the thermal effect of different energy sources on the prostatic tissue. The thermotherapies include transurethral microwave thermoablation (TUMT), interstitial laser coagulation of the prostate (ILCP), high intensity focused ultrasound (HIFU), and transurethral needle ablation (TUNA). Among these treatments, HIFU and TUMT have been used to treat prostate cancer, whereas the other treatments have been thought to be unsuitable for treating cancer.

In HIFU, high intensity precision-focused ultrasound waves are used to heat and destroy targeted prostatic tissue. HIFU is for transrectal use; the tip of the probe is too large to be introduced into the urethra. HIFU can elevate tissue temperature in the focal zone up to 80-100°C in a very short time (1-10 sec) while maintaining the interventional tissue temperature at a safe level. HIFU is considered a contact free "acoustic knife". However, the focal zone of HIFU is restricted to a very small area at a time, it thus takes a very long time (3-8 hours) to cover the entire prostate organ. Furthermore, general anesthesia is usually required. In addition, since the treatment temperature is very high, great care has to be taken when treatment is carried out near the external urethral sphincter or the peripheral prostate, just above the wall of the rectum, to avoid unnecessary tissue damage.

In TUMT, the heat energy is produced by a microwave generator with different microwave frequencies, depending on the machine used. Furthermore, different machines have different designs of the urethral applicator which have different heating profiles. A heating profile that covers substantially the whole prostate is necessary for effective TUMT As a result, not all TUMT machines are suitable for carrying out the present invention. In general, a microwave applicator having its antenna 5-10 mm away from the anchor balloon would be good.

One type of TUMT machine, Urowave (Dornier MedTech), heats and kills cancer cells at 45°C while leaving the adjacent organs outside the fibrous capsule of the prostate unharmed. The heat is delivered to the entire target tissue at one time, allowing the treatment time to be reduced to 1 hour as compared to HIFU. However, the effective depth of microwave using the Urowave machine is limited to about 15mm from the surface of the urethral mucosa, rendering the device ineffective in treating tumors in an enlarged prostate. Therefore, it is advantageous to reduce the volume of the prostate by ADT before Urowave therapy. It is expected that about 90% of the patients with localized prostate cancer, who are candidates for radical prostatectomy or irradiation therapy, can be treated with this less invasive and more selective treatment modality.

There are two primary parameters in a microwave machine that can be adjusted for the purposes of prostate treatment. These are microwave frequency and power. Both change the total energy delivered to the prostate per unit of time. Adjusting power, which is defined as energy loaded per unit time, is a direct method of increasing (or decreasing) the amount of energy delivered. An indirect method of increasing (or decreasing) energy delivered is through the adjustment of frequency. Higher frequency is associated with more energy delivered, but at a lower penetration depth. In the specific case of the Urowave machine, microwave power of 25-90 Watts at frequencies of between 915-1296 MHz can be delivered.

In order to maintain a reasonable treatment time (usually an hour), more power is administered to the larger prostates. However, the amount of power required to effect a treatment is not simply volume-dependent. A prostate with more fibromuscular tissue usually requires more power than one with more glandular tissue. The amount of power administered to each patient also depends on the temperature of the wall of the rectum adjacent to the prostate gland.

In another embodiment, laser thermotherapy is used instead of microwave thermotherapy. In laser thermotherapy, laser light of a specific wavelength is produced by a laser generator. The specific wavelength will depend on the type of laser generator used. The laser generator is connected to a urethral applicator, which acts as an emitter of the laser light. The applicator is inserted transurethrally into the urethra and is kept in place via some physical device, such as an anchor balloon in the bladder. In a preferred embodiment, the urethral applicator emits light in a diffused manner, such that the eventual spherical volume of light radiation encapsulates substantially the whole prostate gland. In a more preferred embodiment, the urethral applicator is incorporated in a transurethral delivery system, which may include a water-cooling circuit system to protect the integrity of non-target tissue. The preferred emitted wavelength is in the range of about 500nm to 2200nm; more preferably in the range of about 700nm to 1600nm; and even more preferably in the range of about 800nm to 1100nm. In a preferred embodiment, the laser power to be applied is in the range of about 10W to 100W and more preferably in the range of about 30W to 60W. The duration of treatment is between 10 minutes to 60 minutes.

### Examples

The following examples further illustrate the present invention:

### Example 1: Treatment of prostate cancer by the combination of androgen deprivation therapy (ADT) and transurethral microwave thermoablation (TUMT)

### Patient Selection

A total of 14 patients with biopsy-proven adenocarcinoma of the prostate, clinical stage T2 or less, during the period between September 2001 and February 2003, were enrolled. Bone scan did not identify metastasis to the bone in all patients. After thorough discussion with each patient, written informed consent was obtained. The mean age of the patients was 71.2 years (range 59-88). The mean serum prostate specific antigen (PSA) was 11.2 ng/ml (range 4.0-30.8). The mean volume of the prostate at initial transrectal ultrasound (TRUS) biopsy was 43.5 ml (range 21.8-85.0). Gleason scores were 6 or less in 7 patients, 7 in 6 patients and 9 in 1 patient. Clinical stages were T1 c in 11 patients and T2a in 3 patients. The patients and tumor characteristics are summarized in Table 1.

### Androgen Deprivation Therapy (ADT)

The therapy started with oral administration of non-steroidal anti-androgen (bicalutamide 80 mg/day in 13 patients and flutamide 375 mg/day in 1 patient) and followed by subcutaneous administration of LH-RH agonist (leuprorelin acetate 3.75 mg/4wks) 2 weeks later. In 4 patients, however, both non-steroidal anti-androgen and LH-RH agonist were started simultaneously, with no evidence of flare-up. Transurethral microwave thermoablation (TUMT) was carried out at least 12 weeks (mean 14.6 weeks) after the initiation of LH-RH agonist so that satisfactory reduction in the volume of the prostate was achieved to render the thermoablation effective. The counting of length of ADT started at the first LH-RH agonist administration and terminated at 6^{th} month (i.e. 24 weeks) after TURP in radical fashion, the final operative procedure.

### Transurethral Microwave Thermoablation (TUMT)

Urowave (Dornier MedTech) was used for TUMT It is a second generation TUMT device, designed for the treatment of BPH *(*Trachtenberg J, Toi A, Yeung E, Habib F. Application of Newer Froms of Therapeutic Energy in Urology. ISIS Medical Media, Oxford, 1995, 51-8*;* Roehrborn CG, Preminger G, Newhall P, et al. Urology, 1998, 51:19-28). It consists of a microwave power generator, a cooling system, and a control and monitoring system. The Urowave applies 915 MHz microwave energy to the prostate via a helical coil antenna enclosed in a transurethral delivery system, which also contains a water-cooling circuit system to provide 360° uninterrupted circumferential cooling to preserve urethral mucosa from heat injury. The diameter of the urethral applicator is approximately 18F when inflated with cooling water. Urethral applicator UA20 is of helical coil antenna of 2 cm in length and UA30 is of 3 cm in length. One can choose an appropriate one according to the length of the prostatic urethra of each patient. Besides, the urethral applicator is equipped with an anchor balloon of 10 ml capacity, approximately 1 cm proximal to the head of the helical antenna to keep the applicator in place during the treatment. In addition, the system features a rectal probe, with a series of 3 thermal couples mounted on the anterior midline (prostate aside) that continuously monitor the temperature at the rectal mucosa closest to the posterior aspect of the prostate. It serves as the key safety feature to prevent excessive heating of the rectal wall.

For the purpose of the present study, the safety threshold was set at 44 °C in the urethral mucosa and at 42.5 °C in the rectum. With these settings, peak intraprostatic temperature reaches as high as 67 °C. Mean temperature reaches a maximum of 55 °C at a radial distance of approximately 0.4 cm from the urethra and remained 45 °C or higher up to a distance of 1.5 cm. Fibrous capsule is a barrier to microwave, but it prevents excessive heating of the rectal wall and external urethral sphincter as well, preserving higher temperature to kill cancer cells inside the prostate. This system is capable of delivering up to 90 watts of power to the targeted tissue. The mean power we used in this series was 48.6 watts (range 25-85).

The patients were subjected to a 60 minutes treatment in an outpatient setting under local anesthesia with lidocaine jelly in the urethra, diclofenac sodium suppository (50 mg) in the rectum and 10 ml of 2% lidocaine hydrochloride infused into the bladder cavity, before the insertion of the urethral applicator for treatment. Indwelling catheter (14 F) was kept in place for 3 days. No patient complained of difficulty in urination after removal of the catheter.

### Observed Reduction in Prostate Volume

Significant reductions in prostatic volume (mean 43.8%) were noted in all patients in 3 months with ADT as listed in Table 2. In another 3 months after TUMT and with continued ADT, reductions in prostate volume (mean 56.5%) was noted as are shown in the same table. These reductions rendered TUMT satisfactorily effective to kill all the cancer cells in the majority of the patients and also made TURP in radical fashion easy and safe. The mean TURP weight of 14 patients was 11.7 grams.

### Histopathological Features of the Therapy

With ADT for 3 months, TRUS biopsy specimens generally revealed progressive degenerative changes, but some fibromuscular hyperplasia with atrophic glands can be seen. In addition, some remnant of adenocarcinoma, though atrophied, but probably viable, can be detected. Three months after TUMT and continued ADT, TURP was carried out in radical fashion and all of the resected chips were subject to thorough histopathological study, which revealed remarkable degeneration with generalized fibrotic changes in the majority of the patients. No cancer cell was detected in 12 of 14 patients.

### Two Patients who had Remnants of Cancer Cell in TURP chips

Case 10 is 67 years old who had a small firm nodule in the left lateral lobe of the prostate by digital rectal examination. Upon TRUS examination, volume of the total gland (TG) of the prostate measured approximately 21.8 ml, transition zone (TZ) measured 7.7 ml, but no hypoechoic lesion was noted in the area where the nodule was felt. In the initial TRUS, 6 systematic biopsy revealed a well differentiated adenocarcinoma, Gleason score 2+2=4, in 2 biopsy cores from the left base and the middle area. Bone scintigraphy with ^{99m}Tc-phosphate did not reveal any metastasis to the bone. Our decision for clinical staging was T2a. After ADT for 14 weeks, the volume of the prostate reduced to 18.7 ml (14.2% reduction) and serum PSA level came down to 3.7 ng/ml (58.4% reduction). The repeated (second) TRUS biopsy at this time revealed atrophic change of the gland; but some well differentiated adenocarcinoma, Gleason score 2+2=4, were seen in biopsy core from the left base. Two weeks after the second TRUS biopsy, TUMT with Urowave using UA20 urethral applicator was carried out for 60 minutes, up to 50 watts. Twelve weeks after the TUMT and continued ADT, TURP in radical fashion was carried out; 7 grams of prostatic tissue were resected from the entire prostatic urethra, divided into 5 parts, median, anterior, left lateral, right lateral and apex. The TRUS measurement of prostatic volume immediately before the TURP was 12.2 ml (44% of original volume). Thorough histopathological study of all the resected chips revealed generalized and significant degenerative changes due to microwave heating throughout almost all the chips; however, a minor cancer lesion, probably viable, remained in a chip from the median bladder neck. The lesion was diagnosed as well differentiated adenocarcinoma, Gleason score 2+3=5. This area where the remaining lesion was detected is right beneath the anchor balloon of the urethral applicator of the Urowave and is a weak point of microwave thermoablation; it is also the easiest point to be thoroughly removed by TURP.

Case 12 is 63 years old who had moderately enlarged smooth-surfaced prostate. Serum PSA level was 6.3 ng/ml. Upon TRUS examination, the prostatic volume (TG) measured 33.6 ml and 6 systematic needle biopsy revealed moderately differentiated adenocarcinoma in 2 biopsy cores. One was a small cancer lesion from a core at the right middle zone, Gleason score of 2+1=3, and another one was in a core from the left apex, Gleason score of 3+4=7. Bone scintigraphy with ^{99m}Tc-phosphate did not reveal any metastasis to bone. The clinical stage was estimated as T1 c. After ADT for 12 weeks, prostatic volume reduced to 18.5 ml (44.9% reduction) and serum PSA level came down to <0.2 ng/ml (96.8% reduction). TUMT with Urowave using UA20 urethral applicator was carried out for 60 minutes, up to 25 watts. Another 13 weeks after TUMT and with continued ADT, TURP in radical fashion was carried out and 5 grams of prostatic tissue were resected from the entire prostatic urethra, divided into 5 parts, median, anterior, left lateral, right lateral and apex. TRUS measurement of prostatic volume immediately before the TURP was 16.0 ml (52.4% reduction). Histopathological study revealed generalized and significant degenerative changes due to microwave heating, throughout almost all the chips. However, a minor cancer lesion, probably viable, was detected in a chip from the right lateral lobe of the prostate. The lesion was diagnosed as moderately differentiated adenocarcinoma, Gleason score 4+3=7. Though this lesion could have been removed by TURP in radical fashion, a second session of TUMT with Urowave using UA20 urethral applicator was carried out for 60 minutes, up to 35 watts, 8 weeks after the TURP Serum PSA level remained at 0.1 ng/ml up to the present, 6 months after termination ADT The patient has been doing well without any clinical evidence of tumor recurrence.

### Example 2: Effect of the Neoadjuvant Hormone Therapy (NHT)

It is generally accepted that NHT reduces preoperative PSA nadir levels and positive margin rates significantly, but does not beneficially decrease the risk of PSA recurrence years after surgery. According to our clinical experience, reduction in prostate volume was very significant (43.8% reduction) after 3 months of ADT The resulting mean volume of 26.2 ml is small enough to be thoroughly heated by TUMT. In 2 patients (no. 9 and no. 14 in Table 2) whose prostate volume at initial examination were large, 85.0 and 73.6 ml, the volumes after 3 months of ADT still measured 47.0 and 45.5 ml, respectively. However, we could not find any cancer cell in their TURP chips, which weighed 31.0 and 24.0 grams, respectively. On the other hand, 2 patients, no. 10 and 12, who showed probably viable cancer cells in TURP chips, had smaller prostates, 12.2 and 16.0 ml, respectively, at TUMT In patient no. 10, cancer cells were found at the base of the prostate close to the median bladder neck which is thought to be least sufficiently heated by TUMT because it is located just beneath the anchor balloon of the urethral applicator and which is the easiest location to be completely removed by TURP. In patient no. 12, probably viable cancer cells were found from TURP chips from the right lateral lobe. It is not entirely clear why these cancer cells survived the TUMT. However, the cancer cells were enclosed by a thick fibrous tissue layer. It is our expectation that all of the remnant cancer cells in these 2 patients were completely removed by the last surgical procedure, the TURP in radical fashion.

**Table 1 Baseline characteristics of patients**

| Case | Age | PSA (ng/ml) | Vol. Prostate (TRUS ml) | Diff. | TRUS-biopsy Gleason score | Location | Clinical stage |
|---|---|---|---|---|---|---|---|
| 1 | 69 | 30.8 | 35.1 | Mod | 4+3=7 | ③⑥ | T1c |
| 2 | 75 | 6,6 | 41.0 | Wel | 2+3=5 | ③ | T1c |
| 3 | 74 | 22.4 | 26.9 | Mod>Por | 4+3=7 | ①②③ | T1c |
| 4 | 88 | 5.2 | 47.0 | Wel | 1+2=3 | ①③ | T1c |
| 5 | 66 | 8.7 | 35,0 | Wel | 2+1=3 | ①⑥ | T1c |
| 6 | 76 | 10.9 | 52.0 | Mod | 4+3=7 | ⑤ | T1c |
| 7 | 59 | 6.8 | 31.9 | Mod | 4+3=7 | ④⑤⑥ | T2a |
| 8 | 76 | 4.0 | 35.9 | Mod | 3+2=5 | ④⑤⑥ | T1c |
| 9 | 71 | 23.0 | 85.0 | Wel>Mod | 3+4=7 | ⑤ | T1c |
| 10 | 67 | 8.9 | 21.8 | Wel | 2+2=4 | ④ | T2a |
| 11 | 81 | 9.2 | 47.8 | Mod>Por | 4+5=9 | ⑤ | T2a |
| 12 | 63 | 6.3 | 33.6 | Mod | 2+1 =3 | ② | T1c |
| | | | | | 3+4=7 | ⑥ | |
| 13 | 75 | 5.0 | 43,6 | Wel | 2+3=5 | ⑤ | T1c |
| 14 | 69 | 9.4 | 73.6 | Mod | 3+3=6 | ③⑥ | T1c |

| | | | | | | | |
|---|---|---|---|---|---|---|---|
| PSA, prostate-specific antigen, Wel, well differentiated. Mod, moderately differentiated, Por, poorly differentiated, TRUS, transrectal ultrasound, ①right base, ②right middle, ③right apex, ④left base, ⑤eft middle, ⑥left apex. | | | | | | | |

**Table 2 Reductions in volume of the prostate measured with transrectal ultrasound by androgen deprivation therapy (ADT) and transurethral microwave thermoablation (TUMT)**

| Case | Before treatment TRUS (ml) | 3 months after ADT TRUS (ml) | 3 months after TUMT TRUS (ml) | TURP wt (g) |
|---|---|---|---|---|
| 1 | 35.1 | - | 14.6 | 5.0 |
| 2 | 41.0 | 24.6 | 21.6 | 16.0 |
| 3 | 26.9 | 21.3 | - | 2.5 |
| 4 | 47.0 | 34.0 | 29.3 | 18.0 |
| 5 | 35.0 | - | 20.2 | 10.1 |
| 6 | 52.0 | 19.4 | 20.0 | 15.0 |
| 7 | 31.9 | 15.4 | 7.4 | 2.8 |
| 8 | 35,9 | 19.6 | 14.6 | 10.2 |
| 9 | 85.0 | 47.0 | 41.7 | 31.0 |
| 10 | 21.8 | 18.7 | 12.2 | 7.0 |
| 11 | 47.8 | 22.5 | 13.6 | 8.0 |
| 12 | 33.6 | 18.5 | 16.0 | 5.0 |
| 13 | 43.6 | 22.5 | 15.7 | 10,0 |
| 14 | 73.6 | 45.5 | 37.0 | 24.0 |
| Mean | 43.6 | 26.2 | 20.8 | 11.7 |
| (range) | (21.8~73.6) | (15.4-47.0) | (7.4-41.7) | (2.8-31.0) |
| Reduction rate 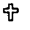 | | -43.8% | -55,5% | |

| | | | | |
|---|---|---|---|---|
| TURP wt, weight of the chips of transurethral resection of the prostate; 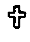 Case 1,3,5 were withdrawn due to insufficiency of data. | | | | |

## Claims

1. Use of a non-steroidal anti-androgen and/or LH, LH-RH or an LH-RH agonist for preparing a medicament for treating cancer, wherein the anti-androgen and/or the agonist are for administration before, simultaneously with, and/or after the application of non-ionizing radiation to the patient.

2. The use according to claim 1, wherein the anti-androgen is selected from bicalutamide or flutamide.

3. The use according to any of claims 1 or 2, wherein the anti-androgen is for administration in a dosage of 10 to 1000 mg/day.

4. The use according to any of claims 1 to 3, wherein the agonist is selected from leuprorelin acetate, goserelin acetate, buserelin acetate and tripterelin.

5. The use according to any of claims 1 to 4, wherein the agonist is for administration in a dosage of 1-10 mg/4 weeks.

6. The use according to any of claims 1 to 5, wherein the cancer is prostate cancer.

7. The use according to any of claims 1 to 6, wherein the prostate cancer is an adenocarcinoma.

8. The use according to any of claims 1 to 7, wherein the anti-androgen and the agonist are for simultaneous or sequential administration.

9. The use according to any of claims 1 to 8, wherein the anti-androgen is for oral administration and the agonist is for subcutaneous administration.

10. The use according to any of claims 1 to 9, wherein the application of non-ionizing radiation is started at least 4 weeks after the start of the administration of the anti-androgen and/or the agonist.

11. The use according to claim 10, wherein the application of non-ionizing radiation is started 3 months after the start of the administration of the anti-androgen and/or the agonist.

12. The use according to any of claims 1 to 11, wherein the non-ionizing radiation is to be applied simultaneously with the ongoing administration of the anti-androgen and/or the agonist.

13. The use according to any of claims 1 to 12, wherein the administration of the anti-androgen and/or the agonist continues after the non-ionizing radiation for a period of at least 1 month.

14. The use according to claim 13, wherein the administration of the anti-androgen and/or the agonist continues after the non-ionizing radiation for a period of 3 months.

15. The use according to any of claims 1 to 14, wherein the non-ionizing radiation is heat-generating radiation.

16. The use according to claim 15, wherein the heat-generating radiation is selected from microwave, laser light and ultrasound.

17. The use according to claim 16, wherein the heat-generating radiation is applied as microwave energy with a frequency range between 800 MHz to 1500 MHz.

18. The use according to claim 17, wherein the frequency range is between 800 MHz and 1300 MHz.

19. The use according to claim 17 or 18, wherein the frequency range is between 800 MHz and 1000 MHz.

20. The use according to any of claims 17 to 19, wherein microwave radiation in a power range of 25 to 100 Watts is to be applied to the targeted tissue.

21. The use according to claim 20, wherein the power range is between 25 to 90 Watts.

22. The use according to claim 20 or 21, wherein the power range is between 25 to 85 Watts.

23. The use according to any of claims 17 to 22, wherein the non-ionizing heat-generating microwave radiation is to be applied by way of transurethral microwave thermoablation (TUMT).

24. The use according to any of claims 17 to 22, wherein the non-ionizing heat-generating microwave radiation is applied for a period of between 10 minutes and 60 minutes.

25. The use according to any of claims 15 to 24, wherein the heat-generating radiation is applied at a time of at least 1 month after the start of the anti-androgen and/or agonist administration, preferably at least 3 months after the start of the anti-androgen and/or agonist administration, and more preferably simultaneously to the ongoing administration of the anti-androgen and/or the agonist.

26. The use according to claim 16, wherein the heat-generating radiation is applied as laser radiation with a wavelength range of between 500 nm and 2200 nm.

27. The use according to claim 26, wherein the wavelength range is between 700 nm and 1600 nm.

28. The use according to claim 26, wherein the wavelength range is between 800 nm and 1100 nm.

29. The use according to any of claims 26 to 28, wherein a laser power in the range of 10W to 100W is applied to the targeted tissue.

30. The use according to claim 29, wherein the laser power range is between 30W and 60W.

31. The use according to any of claims 26 to 30, wherein the laser radiation is applied for a period of between 10 minutes and 60 minutes.

32. A therapeutic combination for treating cancer, preferably prostate cancer, comprising:
(a) a non-steroidal anti-androgen, and/or
(b) LH, LH-RH, or a LH-RH agonist, and
(c) non-ionizing radiation,
wherein the androgen and/or the agonist are for administration before, simultaneously with, and/or after the application of radiation to the patient.
